# EUROPEAN PATENT APPLICATION

(11) **EP 3 599 246 A1**
(43) Date of publication of application: **29.01.2020**
(21) Application number: 18185429.0
(22) Date of filing: 25.07.2018
(51) Int. Cl.: C07K 14/00

(54) **MEANS AND METHODS FOR SYNTHESIZING PRECURSORS OF Y-AMINOBUTYRIC ACID (GABA) ANALOGS**

(71) Applicant: RIJKSUNIVERSITEIT GRONINGEN, 9712 CP Groningen (NL)
(72) Inventor: POELARENDS, Gerrit Jan, 9713 AV Groningen (NL); BIEWENGA, Lieuwe, 9713 AV Groningen (NL); VAN DER MEER, Jan-Ytzen, 9713 AV Groningen (NL); PODDAR, Harshwardhan, 9713 AV Groningen (NL); THANGAVELU, Saravanan, 9713 AV Groningen (NL)
(74) Representative: V.O.

(57) **Abstract**

The invention relates to the fields of drug development and biocatalysis, more specifically to a biocatalytic route for asymmetric synthesis of precursors of y-aminobutyric acid (GABA) analogs. Provided is an isolated mutant 4-oxalocrototonate tautomerase (4-OT) enzyme comprising the following mutations (i) leucine at position 8 substituted with a tyrosine (L8Y) or a phenylalanine (L8F); (ii) methionine at position 45 substituted with a tyrosine (M45Y); and (iii) phenylalanine at position 50 substituted with an alanine (F50A), wherein the positions are numbered according to the amino acid sequence of 4-OT of *Pseudomonas putida.* Also provided is a method for the synthesis of a precursor for the pharmaceutically relevant enantiomer of a GABA analog, comprising (i) providing a y-nitroaldehyde using the 4-OT mutant enzyme, followed by (ii) subjecting the thus obtained y-nitroaldehyde to an enzymatic oxidation reaction catalyzed by an aldehyde dehydrogenase (EC 1.2.1.3).

## Description

The invention relates to the fields of drug development and biocatalysis. More specifically, it relates to a biocatalytic route for asymmetric synthesis of both enantiomers of γ-nitrocarboxylic acids, which are practical precursors of γ-aminobutyric acid (GABA) analogs.

GABA analogs form an important class of pharmaceuticals. With an increasing world population and life expectancy, the demand for GABA analogs is expected to increase. However, the synthesis of GABA analogs can be challenging and synthesis routes described so far often involve kinetic resolutions with low atom efficiencies ¹⁻³. Therefore, it is highly desirable to investigate alternative synthesis routes that are potentially "greener" and more efficient.

An alternative synthesis route that has been investigated comprehensively is the asymmetric Michael-type addition of aldehydes to α,β-unsaturated nitroalkenes to form γ-nitroaldehydes. In two chemical steps (oxidation of the aldehyde and reduction of the nitro functionality) γ-nitroaldehydes can be converted into the desired GABA analogs⁴. Multiple organocatalytic approaches to obtain enantioenriched γ-nitroaldehydes have been reported (reviewed in 5). Also, small proline derived peptides have been utilized for this type of catalysis⁶⁻⁹. However, most organo- and peptide catalysts require environmentally unfriendly solvents, high catalyst loadings and do not reach the high enantioselectivity required for application in the production of medicines. Although the use of organocatalysts in aqueous solutions has been demonstrated, this requires extensive remodeling of the catalyst, making it more difficult to obtain the final catalyst^{10,11}.

Unlike organocatalytic approaches, only a very limited number of studies which use enzymes for this type of synthesis has been reported in the literature. In fact, only a few enzymes are known to be able to catalyze any type of C-C bond-forming Michael addition¹². Recently, Hilvert and co-workers published the elegant enzymatic synthesis of γ-nitroketones, but not γ-nitroaldehydes, by both acetone addition to nitroalkenes, and nitroalkane addition to conjugated ketones¹³. However, the obtained γ-nitroketones cannot be converted into GABA analogs in a straight forward manner. Since acetaldehyde could not be used as a substrate in additions to nitroalkenes, the reported enzyme can not be used for the synthesis of γ-nitroaldehydes.

The present inventors previously reported on a small tautomerase, 4-oxalocrotonate tautomerase (4-OT) from *Pseudomonas putida,* that can promiscuously catalyze the addition of small aldehydes, most notably the highly reactive acetaldehyde, to aryl- or alkyl- substituted nitroalkenes¹⁴⁻¹⁶. The natural reaction that is catalyzed by 4-OT is the tautomerization of 2-hydroxymuconate to 2-oxohex-3-enedioate, in which Pro-1, the key catalytic residue of 4-OT, acts as a general base^{17,18}. Analogous to proline-based organocatalysts, Pro-1 of 4-OT is also the key catalytic residue in many promiscuous activities including aldol condensations^{19,20} and Michael-type additions¹⁴, most likely via an enamine intermediate^{20,21}. By systematic mutagenesis, 4-OT mutants were identified that show an increased catalytic rate, increased enantioselectivity or with inverted enantioselectivity²². However, for the synthesis of the pharmaceutically relevant enantiomer of GABA analogs, the reported enantiomeric ratios (e.r.) are only modest.

Therefore, the inventors set out to develop a novel biocatalytic synthesis route for the highly enantioselective synthesis of several GABA analogs, like pregabalin, baclofen and phenibut. They specifically aimed at providing a highly enantioselective, solvent stable artificial enzyme, which shows very high conversions under aqueous conditions, with a high (at least 60%) final yield and an enantiomeric ratio of the desired product of 95:5 and higher.

At least some of these goals were met by the provision of genetically engineered point mutants of 4-OT, herein after also referred to as 'artificial Michaelases', showing a significantly improved catalytic rate.

By employing an artificial Michaelase together with a natural aldehyde dehydrogenase and a NADH-oxidase for cofactor recycling in a one-pot two-step cascade, γ-nitrocarboxylic acids could be efficiently synthesized with short reaction times, high yields and excellent enantioselectivity. Finally, the γ-nitrocarboxylic acids could be reduced to yield the final GABA analogs in a three-step one-pot cascade. All three steps were performed under aqueous conditions with very high conversions, with excellent final isolated yields of 64% up to 74% and e.r.'s of 98:2 and higher. This synthetic route highlights the exciting opportunities available for combining designed artificial biocatalysts, natural enzymes, and chemocatalysts, in multistep syntheses. Moreover, the present invention demonstrates the power of combining different types of catalysts in multi-step reactions, to overcome difficulties of particular classes of catalysts, and to facilitate the development of an economically feasible greener synthesis route towards GABA-analogs.

Accordingly, in one embodiment the invention provides an isolated mutant 4-oxalocrototonate tautomerase (4-OT) enzyme comprising the following mutations : (i) leucine at position 8 substituted with a tyrosine (L8Y) or a phenylalanine (L8F); (ii) methionine at position 45 substituted with a tyrosine (M45Y); and (iii) phenylalanine at position 50 substituted with an alanine (F50A), wherein the positions are numbered according to the amino acid sequence of 4-OT of *Pseudomonas putida.*

4-Oxalocrotonate tautomerase (EC 5.3.2.-4-OT) is an enzyme that converts 2-hydroxymuconate to the α,β-unsaturated ketone, 2-oxo-3-hexenedioate. This enzyme forms part of a bacterial metabolic pathway that oxidatively catabolizes toluene, o-xylene, 3-ethyltoluene, and 1,2,4-trimethylbenzene into intermediates of the citric acid cycle. With a monomer size of just 62 amino acid residues, 4-OT is one of the smallest enzymes known. However, in solution, the enzyme forms a hexamer of six identical subunits, so the active site is formed by amino acid residues from several subunits. The 4-OT enzyme is also unusual in that it uses a proline residue at the amino terminus as an active site catalytic residue. In addition to Pro-1, three other residues (Arg-11, Arg-39, and Phe-50) have been identified as critical catalytic residues by kinetic analysis, site-directed mutagenesis, chemical synthesis, NMR, and crystallographic studies. Arginine-39 functions as the general acid catalyst (assisted by an ordered water molecule) in the reaction while Arg-11 plays a role in substrate binding and facilitates catalysis by acting as an electron sink. Finally, the hydrophobic nature of the active site, which lowers the pKa of Pro-1 to ∼6.4 and provides a favorable environment for catalysis, is largely maintained by Phe-50.

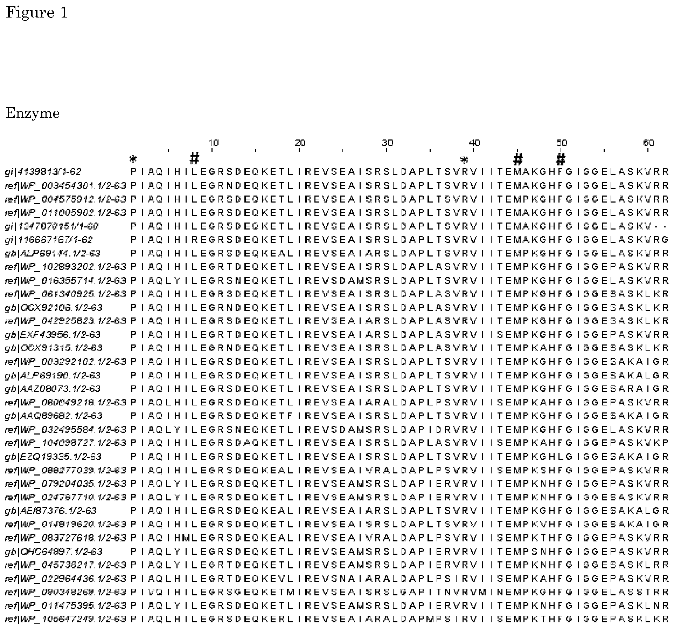

A mutant 4-OT enzyme of the invention can be based on a 4-oxalocrotonate tautomerase polypeptide known in the art. See Figure 1 showing exemplary 4-OT enzymes derived from various micro-organisms defined by their NCBI accession number. Each of these and further "natural" 4-OT polypeptides can serve as parent enzyme from which the mutant of the invention is derived. It is to be understood that the sequence of a mutant enzyme, as compared to the parent sequence, may comprise one or more amino acid alterations additional to the above mentioned specific substitutions at positions 8, 45 and 50, provided that the desired enzyme functionality is maintained.

A mutant 4-OT enzyme provided herein is, among others, characterized by an inverted and increased enantioselectivity as compared to wild-type 4-OT. In one embodiment, it catalyzes the addition of **1** to **2a** 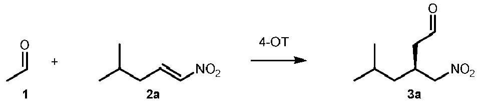 to obtain product *S*-**3a,** the pharmaceutically relevant enantiomer of the GABA-analog pregabalin precursor, with an enantiomeric ratio of at least 95:5, preferably at least 97:3, more preferably at least 98:2.

Alternatively or additionally, the mutant enzyme catalyzes the addition of **1** to **2b** 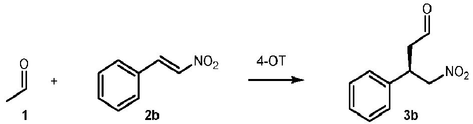 to obtain product *R*-**3b** (the pharmaceutically relevant enantiomer of the GABA-analog phenibut precursor), with an enantiomeric ratio of at least 95:5, preferably at least 97:3 more preferably at least 98:2.

Alternatively or additionally, it catalyzes the addition of **1** to **2c** 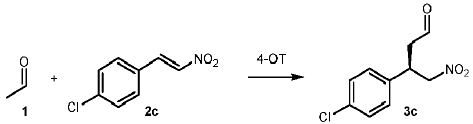 to obtain product *R***-3c** (the pharmaceutically relevant enantiomer of the GABA-analog baclofen precursor), with an enantiomeric ratio of at least 95:5, preferably at least 97:3.

Alternatively or additionally, it catalyzes the addition of **1** to **2d** 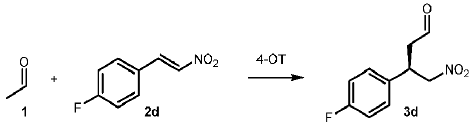 to obtain product *R*-**3d** (the pharmaceutically relevant enantiomer of the GABA-analog p-F-phenibut (known as fluorophenibut) precursor), with an enantiomeric ratio of at least 95:5, preferably at least 97:3.

Preferably, residues or motifs known to be conserved among the family of 4-OT enzymes are not modified or replaced with (stretches of) chemically similar amino acids, also known in the art as conservative substitutions. See Figure 1 of the present application indicating conserved (catalytic) residues, and Figure 3b in ref.22 showing the mutability landscape of 4-OT for the reaction between **1** and **2b** shown herein above. In one embodiment, the mutant 4-OT contains the conserved catalytic residues Pro-1, Arg-11 and Arg-39 that are involved in the desired Michael-type addition activity, and optionally one or more residues that are conserved, such as Gly-10 and Gly-54.

For example, the mutant 4-OT enzyme contains one or more conservative amino acids substitution(s). Substitution tables providing functionally similar natural amino acids are well known in the art. Such conservatively modified variants are in addition to the specific set of three mutations as described herein.

In one embodiment, a conservative substitution is based on the following eight groups which each contain amino acids that are conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M)

In another embodiment, a conservative substitution is a substitution in which one amino acid within the following groups (a) - (e) is substituted by another amino acid residue within the same group: (a) small aliphatic, nonpolar or slightly polar residues: Ala, Ser, Thr, Pro and Gly; (b) polar, negatively charged residues and their (uncharged) amides: Asp, Asn, Glu and Gln; (c) polar, positively charged residues: His, Arg and Lys; (d) large aliphatic, nonpolar residues: Met, Leu, Ile, Val and Cys; and (e) aromatic residues: Phe, Tyr and Trp.

Particularly preferred conservative substitutions are as follows: Ala into Gly or into Ser; Arg into Lys; Asn into Gln or into His; Asp into Glu; Cys into Ser; Gln into Asn; Glu into Asp; Gly into Ala or into Pro; His into Asn or into Gln; Ile into Leu or into Val; Leu into Ile or into Val; Lys into Arg, into Gln or into Glu; Met into Leu, into Tyr or into Ile; Phe into Met, into Leu or into Tyr; Ser into Thr; Thr into Ser; Trp into Tyr; Tyr into Trp; and/or Phe into Val, into Ile or into Leu.

In one embodiment, the invention provides a mutant 4-OT which shows at least 80%, preferably at least 85%, more preferably at least 90% sequence identity to the amino acid sequence P**I**AQIHI(**Y/F**)EGRSDEQKETLIREVSEAISRSLDAPLTSVRVIITE**Y**AKGH**A**GIGG ELASKVRR, provided that it comprises the residues Y/F,Y and A indicated in bold at, respectively, positions 8, 45 and 50.

In a further embodiment, the mutant enzyme shows at least 93%, 94%, 95%, 96%, 97%, 98% or at least 99% sequence identity to the amino acid sequence **PI**AQIHI(**Y/F**)E**GR**SDEQKETLIREVSEAISRSLDAPLTSV**R**VIITE**Y**AKGH**A**GIG**G** ELASKVRR, provided that it comprises the residues Y/F,Y and A indicated in bold at, respectively, positions 8, 45 and 50.
In a still further embodiment, the mutant enzyme shows at least 93%, 94%, 95%, 96%, 97%, 98% or at least 99% sequence identity to the amino acid sequence **P**IAQIHI(**Y/F**)E**GR**SDEQKETLIREVSEAISRSLDAPLTSV**R**VIITE**Y**AKGHAG**I**G**G** ELASKVRR, provided that it comprises the residues indicated in bold.

In a still further embodiment, the mutant enzyme shows at least 93%, 94%, 95%, 96%, 97%, 98% or at least 99% sequence identity to the amino acid sequence **PIAQ**IH**I(Y/F)EGR**S**DEQKETLIREVSEA**IS**RSLD**A**P**LTS**VRVII**T**EY**A**K**G**HAGI GGE**L**A**SKVR**R**, provided that it comprises the residues indicated in bold.

In another embodiment, the mutant enzyme has the amino acid sequence

PI(A/V)Q(I/L)(H/Y)(I/M)(Y/F)EGR(S/T/N)(D/N/G)(E/A)QKE(T/A/V/R)(L/F/M)IREVS
(E/D/N)A(I/M)(S/V/A)R(S/A)L(D/G)AP(L/I/M)(T/A/P/E/D)(S/R/N)(V/I)RV(I/M)I
(T/S/N)Y(A/P)(K/S)(G/A/S/N/V/T/A)HAGIGGE(L/P/S)A(S/K/R)(K/A/S)(V/I/L/T)(R/K/
G/N/-)(R/G/P/-)

In yet another embodiment, the mutant enzyme has the amino acid sequence

PIAQ(I/L)(H/Y)I(Y/F)EGR(S/T/N)DEQKETLIREVSEA(I/M)(S/V/A)RSLDAP(L/I)(T/
A/P/E)(S/R)VRVII(T/S)EY(A/P)K(G/A)HAGIGGE(L/P/S)A(S/K)(K/A)(V/I/L)(R/K/G)R
.

It was surprisingly observed that both the mutant L8Y/M45Y/F50A and the mutant L8F/M45Y/F50A exhibited an excellent enantioselectivity. Also, they display an increased catalytic rate as compared to the double mutant M45Y/F50A known in the art. However, mutant L8Y/M45Y/F50A of the invention was found to have the highest catalytic rate of the mutants tested. Moreover, this triple mutant proved to be much more stable in the presence of alcohol, tolerating ethanol concentrations of up to 30 vol%.

Accordingly, in a preferred embodiment the mutant 4-OT of the invention comprises the mutations L8Y, M45Y and F50A.

A further embodiment of the invention relates to a nucleic acid molecule that encodes a mutant 4-OT enzyme according to the invention. Degeneracy of a codon is also considered. Also provided is a recombinant vector that comprises the nucleic acid molecule. The vector is capable of providing the mutant enzyme when it is expressed, and includes, as a matter of course of a nucleic acid having a base sequence corresponding to the amino acid sequence of the present mutant enzyme.

Also provided is a recombinant host cell transformed with the vector, such as a bacterial, fungal or yeast host cell. In a preferred embodiment, the host cell is a bacterial cell.

The embodiments herein above provide a unique biocatalytic approach to synthesize these γ-nitroaldehydes using a highly enantioselective artificial Michaelase enzyme (i.e. a mutant 4-OT comprising at least the set of three specific mutations) that is highly selective towards the pharmaceutically relevant enantiomer. Accordingly, the invention provides a method for the biocatalytic synthesis of a γ-nitroaldehyde via a Michael-type addition of an aldehyde to an α,β-unsaturated nitroalkene, comprising the use of a mutant 4-OT enzyme, a nucleic acid, a vector and/or a host cell according to the invention.

The biocatalytic synthesis typically comprises reacting the aldehyde and the α,β-unsaturated nitroalkene in the presence of a mutant 4-OT enzyme according to the invention. The mutant may be used as crude enzyme, e.g. as a cell free extract or a semi-purified or purified enzyme. The aldehyde is preferably used in molar excess of the nitroalkene reactant. For example, 30-200 mM of aldehyde is reacted with 1-10 mM α,β-unsaturated nitroalkene. The amount of enzyme is not critical but generally lies in the range of about 1-10 mol%, preferably 2-6 mol%, relative to the concentration of the nitroalkene reactant in the reaction mixture. In one embodiment, mutant 4-OT is used at a concentration of 10-130 µM, preferably 20-100 µM.

The Michael-type addition reaction is advantageously performed in an aqueous reaction buffer and a co-solvent. Typically, the co-solvent improves substrate solubility. In particular, it is a water miscible co-solvent that allows higher nitroalkene substrate loadings.

Exemplary co-solvents include DMSO, suitably used in the range of 1 to 40 vol%, and linear alcohols, like methanol and ethanol, or diols, like ethylene glycol, 1,3-propanediol and 1,4-butanediol) that are suitably used in the range of 5 to 30 vol%. The reaction buffer may have a pH in the range of 5-8, preferably at about pH 6-7. Suitable buffers include sodium phosphate, sodium citrate, and triethanolamine (TEA). In a preferred embodiment, the reaction mixture comprises 20 mM sodium phosphate buffer pH 6.5, 20-30 vol% EtOH. Whereas the reaction is advantageously performed at room temperature, the reaction temperature can be below or above room temperature, like in the range of about 10-30°C. Lower reaction temperatures are also encompassed possible; depending on the co-solvent even below 0°C is possible.

The skilled person will understand that the reaction time can vary according to the reactants, the specific reaction conditions, the desired yield, etc. Good conversions were already observed within 60 minutes.

The inventors furthermore recognized that the engineered artificial Michaelases of the invention are advantageously combined with a natural aldehyde dehydrogenase to provide an enzymatic two-step cascade to synthesize γ-nitrocarboxylic acids. Accordingly, in one embodiment the invention provides a two-step method for the enzymatic synthesis of a precursor for the pharmaceutically relevant enantiomer of a GABA analog, comprising (i) providing a γ-nitroaldehyde using a mutant 4-OT enzyme of the invention, followed by (ii) subjecting the thus obtained γ-nitroaldehyde to an enzymatic oxidation reaction catalyzed by an aldehyde dehydrogenase (EC 1.2.1.3), to obtain the corresponding γ-nitrocarboxylic acid.

It was found that this oxidation reaction is not dependent on nor limited to the enzymatic conversion catalyzed by one of the specific 4-OT enzymes of the invention; the modularity of the reaction is for example demonstrated by using a previously engineered enantiocomplementary 4-OT mutant, 4-OT A33D, to synthesize the other enantiomer of γ-nitrocarboxylic acids. Accordingly, in one embodiment the invention provides a method for the enzymatic synthesis of a compound having a γ-nitrocarboxylic acid functionality, comprising subjecting a compound having a γ-nitroaldehyde functionality to an enzymatic oxidation reaction catalyzed by an aldehyde dehydrogenase (EC 1.2.1.3), to obtain the corresponding compound having a γ-nitrocarboxylic acid functionality.

Aldehyde dehydrogenases (EC 1.2.1.3) are a group of enzymes that catalyse the oxidation of aldehydes. Despite the name "dehydrogenase", their mode of oxidation is by addition of oxygen rather than by removal of hydrogen - that is, they convert aldehydes (R-C(=O)-H) to carboxylic acids (R-C(=O)-O-H).

This enzyme belongs to the family of oxidoreductases, specifically those acting on the aldehyde or oxo group of donor with NAD⁺ or NADP⁺ as acceptor. The systematic name of this enzyme class is aldehyde:NAD⁺ oxidoreductase. Other names in common use include CoA-independent aldehyde dehydrogenase, m-methylbenzaldehyde dehydrogenase, NAD-aldehyde dehydrogenase, NAD-dependent 4-hydroxynonenal dehydrogenase, NAD-dependent aldehyde dehydrogenase, NAD-linked aldehyde dehydrogenase, propionaldehyde dehydrogenase, and aldehyde dehydrogenase (NAD).

Suitable aldehyde dehydrogenases for use in the present invention are known in the art, and can be obtained from commercial sources. For example, in one embodiment the aldehyde dehydrogenase is the enzyme sold as crude cell free extract by Prozomix Ltd. (Haltwhistle, UK) under the tradename PRO-ALDH(003).

The enzymatic oxidation reaction is advantageously performed under conditions wherein the co-factor for the aldehyde dehydrogenase (NAD⁺ or NADP⁺ ) is recycled. In general, cofactor recycling can be achieved by coupling a desired enzymatic reaction with an additional chemical, electrochemical, photocatalytic, or enzymatic reaction, and the enzymatic method is favored. In one embodiment, the enzymatic oxidation reaction is performed in the presence of NAD⁺ and the co-factor regenerator is an NADH oxidase (EC 1.6.3.1). Suitable NADH oxidases for use in the present invention are known in the art, and can be obtained from commercial sources. For example, in one embodiment the NADH oxidase is the enzyme sold as crude cell free extract by Prozomix Ltd. (Haltwhistle, UK) under the tradename PRO-NOX(009), which is strictly NADH dependent.

In another embodiment, the enzymatic oxidation reaction is performed in the presence of NADP⁺ and the co-factor regenerator is an NADPH oxidase (EC 1.6.3.1). Alternatively, an NADH oxidase is used that also accepts NADPH, for example an oxidase from *Lactobacillus sanfranciscensis* described by Riebel et al. (2003), Adv. Synth. Catal., 345: 707-712).

The amount of enzyme(s) to be used in the second step of the enzymatic cascade to synthesize γ-nitrocarboxylic acids can be determined by routine optimization. For example, the reaction mixture comprises about 0.1- 2 mg/mL aldehyde dehydrogenase and about 0.2- 2 mg/mL NADH oxidase. Preferably, the co-factor regenerator is used in excess over the aldehyde dehydrogenase. Good results can be obtained with a reaction mixture comprising about 0.2- 1 mg/mL aldehyde dehydrogenase and about 0.5- 1.5 mg/mL NADH oxidase.

The two-step enzymatic method according to the invention to synthesize a γ-nitrocarboxylic acid by combining of different types of catalysts is advantageously performed as two consecutive steps in a one-pot process. Exemplary embodiments of the two-step one-pot process are summarized in Table 3.

To go a step further, the inventors observed not only that the enantiopure γ-nitrocarboxylic acids obtained by the newly engineered two-step enzymatic cascade process are more stable and practical GABA precursors, but that they are readily converted to pharmaceutically active GABAs by only one simple chemical step. More in particular, this step involves the chemical reduction of the nitro-moiety of the γ-nitrocarboxylic acid to the corresponding amine.

Accordingly, in one embodiment the invention provides a method for the synthesis of a precursor for the pharmaceutically relevant enantiomer of a GABA analog, comprising (i) providing a γ-nitroaldehyde using a 4-OT mutant enzyme according the invention, followed by (ii) subjecting the thus obtained γ-nitroaldehyde to an enzymatic oxidation reaction catalyzed by an aldehyde dehydrogenase (EC 1.2.1.3) to obtain the corresponding γ-nitrocarboxylic acid; and (iii) of chemically reducing the nitro-moiety of the γ-nitrocarboxylic acid to obtain a GABA analog.

The chemical reduction is preferably performed using a reducing agent, for example NaBH₄ (sodium borohydride) in combination with a transition metal halide or salt. Other catalysts known for reduction of nitro groups may also be used, such as Ra-Ni or Pd/C in the presence of a hydrogen source, but these require high organic solvent/water ratios. Preferably, NaBH₄ is used in combination with a Ni-based catalyst, such as NiCl₂.6H₂O or Ni(OAc)₂.4H₂O. In a specific aspect, step (iii) comprises the use of nickel boride (NaBH₄/NiCl₂.6H₂O) as catalyst.

The reaction conditions for the reduction reaction are known to a person skilled in the art. Preferably, reduction of the nitro-moiety of the γ-nitrocarboxylic acid is performed at room temperature in an aqueous solution at pH 4 or lower, e.g. pH 1-4.

Moreover, it could be demonstrated that this required chemical step can be combined with the two enzymatic steps in one pot to produce pharmaceutically active GABAs out of simple starting materials (acetaldehyde and nitroalkenes) and avoiding (de-)protecting steps and intermediate purification. Accordingly, in a preferred embodiment, steps (i), (ii) and (iii) are performed as a three-step one-pot process. Table 4 provides exemplary embodiments of a three-step one-pot chemoenzymatic synthesis of GABA analogs.

The novel methodology provided herein thus includes the design and application of a new biocatalyst for the asymmetric Michael-type addition of acetaldehyde to nitroolefin acceptors, providing convenient access to enantiopure γ-nitroaldehydes, which are practical precursors for pharmaceutically active GABAs.

For example, a 4-OT mutant and/or method of the invention is advantageously used in the synthesis of a precursor of a GABA analog selected from the group consisting of pregabalin, baclofen, rolipram, phenibut and para-F-phenibut.

### LEGEND TO THE FIGURES

**Figure 1****:** Multiple sequence alignment of 4-OT from *Pseudomonas putida* mt-2 (top line) with homologous enzyme sequences (indicated by NCBI accession number) showing at least 80% sequence identity, each of which can be used as parent enzyme for obtaining a mutant 4-OT enzyme of the invention. Indicated with * are residues Pro-1 and Arg-39, which are essential for catalysis. Indicated with # are positions that are mutated to give an 4-OT enzyme of the invention having an inverted and increased enantioselectivity.

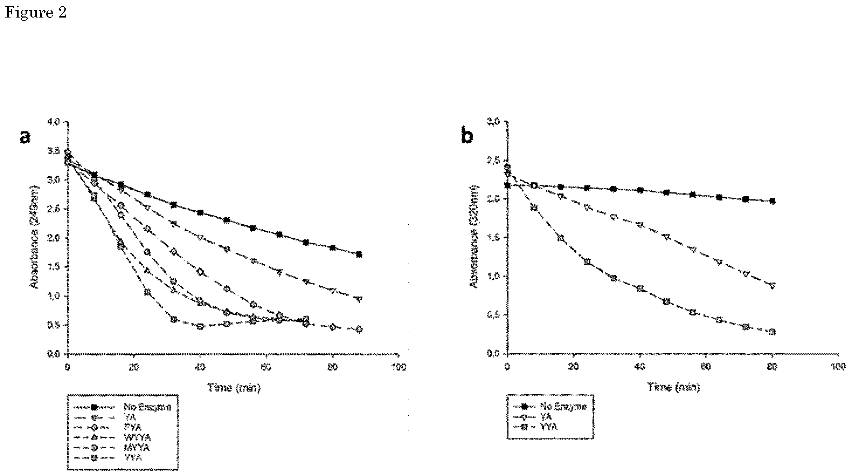

**Figure 2****: 4-OT catalyzed Michael-type additions.** (Panel A) Addition of **1** (150 mM) to **2a** (5 mM) in 20 mM sodium phosphate buffer pH 6.5 and 5% v/v DMSO. Different 4-OT mutants were used as a catalyst at a concentration of 100 µM. YA: 4-OT M45Y/F50A, FYA: 4-OT L8F/M45Y/F50A, WYYA: 4-OT I2W/L8Y/M45Y/F50A, MYYA: 4-OT I2M/L8Y/M45Y/F50A, YYA: 4-OT L8Y/M45Y/F50A. (Panel B) Addition of 1 (65 mM) to **2c** (1.3 mM) in 20 mM sodium phosphate buffer pH 6.5 and 45% v/v DMSO. Different 4-OT mutants were used as a catalyst at a concentration of 18 µM. YA: 4-OT M45Y/F50A, YYA: 4-OT L8Y/M45Y/F50A.

### EXPERIMENTAL SECTION

### Example 1: Engineering of an enantioselective Michaelase

In earlier research, the construction and the catalytic performance of 4-OT mutant M45Y/F50A was reported²². Introduction of these mutations inverted the enantioselectivity of 4-OT, thereby allowing the enzymatic synthesis of *S*-**3a** and *R-***3b-d,** which include the valuable pharmaceutically relevant enantiomers of precursors of the GABA-analogs pregabalin (**3a**), phenibut (**3b**) and baclofen (**3c**) (Scheme 1).

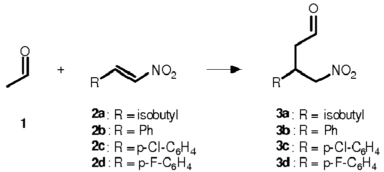

Scheme 1: 4-OT catalyzed Michael-type additions. Synthesis of γ-nitroaldehydes via a Michael-type addition of acetaldehyde to α,β-unsaturated nitroalkenes.

However, the enantioselectivity of 4-OT M45Y/F50A is moderate, with for instance an e.r. of 90:10 for **3a,** the precursor of pregabalin, and only 62:38 for **3c,** the precursor of baclofen.

In order to improve the catalyst in terms of enantioselectivity and catalytic rate, the present inventors constructed a Combinatorial Active-site Saturation Test (CAST) library. This library was constructed by randomizing positions Ile-2 and Leu-8 in the context of mutant M45Y/F50A (I2X/L8X/M45Y/F50A).

### Mutant library construction

The 4-OT I2X/L8X/M45Y/F50A library was constructed by Quikchange technology. The following primers were used: Fw 5'-GGA GAT ATA CAT ATG CCT **NNK** GCC CAG ATC CAC ATC **NNK** GAA GGC CGC AGC G-3' and Rv 5'-C GCT GCG GCC TTC **MNN** GAT GTG GAT CTG GGC **MNN** AGG CAT ATG TAT ATC TCC-3'. The mutated codons are indicated in bold. 40 ng of pJexpress 414 plasmid DNA containing the 4-OT M45Y/F50A gene was used as template²². The PCR reaction was conducted using Phusion polymerase (New England Biolabs) in 1x HF buffer (New England Biolabs) in 50 µl reaction volume. The following PCR program was used: 98 °C, 10min (Initial denaturation), followed by 18 cycles of 95 °C 30 s, 55 °C 1 min, 68 °C 4 min and a final elongation step 68 °C 10min. The reaction mixtures were 2x diluted with water followed by the addition of 1 µl FastDigest DpnI (ThermoFisher Scientific) to digest the template DNA. Reaction mixtures were placed at 37 °C for 2 h, followed by a DpnI denaturing step of 80 °C for 10 min. An initial transformation in Dh5α was performed and the plasmid DNA of individual colonies was isolated and sequenced to confirm the library quality. 4 parallel transformations were performed using 50 µl of 10-beta competent E. coli (High efficiency, New England Biolabs) and 5 µl of the PCR reaction mixture. Transformants were selected on LB-agar plates supplemented with 100 µg/mL ampicillin. ∼8000 colonies were resuspended in 10 mM sodium phosphate buffer and plasmid DNA was isolated and subsequently used to transform E. coli B121(DE3) cells for expression of the 4-OT mutants.

### Library screening

After transformation with 4-OT I2X/L8X/M45Y/F50A library DNA, individual E. coli B121(DE3) colonies were used to inoculate 2x 1.25 mL LB supplemented with 100 µg/mL ampicillin and 100 µM Isopropyl β-D-1-thiogalactopyranoside (IPTG) in 96-deep well plates (Greiner Bio-one, 96-well Masterblock). The plates were sealed with sterile gas-permeable seals (Greiner Bio-one, BREATHseal) and incubated at 37 °C, overnight shaking at 250 rpm. After the incubation, the plates were centrifuged (3500 rpm, 8 min). The supernatant was discarded and the individual pellets were lysed by resuspension in 350 µl BugBuster (Novagen) supplemented with 25 U/mL benzonase (Novagen). The lysis was continued for 20 minutes at room temperature under vigorous shaking. The lysates were cleared by centrifugation (3500 rpm, 55 min, 4 °C) after which the Cell Free Extract (CFE) was obtained. The final reaction mixture for the addition of 1 to 2a consisted of the following: CFE (40% v/v), 150 mM 1, 5 mM 2a, DMSO (5% v/v) in 20 mM sodium phosphate buffer pH 6.5, 500 µl final volume. The reactions were performed in 96-deep well plates sealed by ultraviolet transparent plate seals (VIEWseal, Greiner Bio-One) at room temperature. After 50 minutes the reaction was stopped by extraction with 300 µl toluene, which caused the proteins to precipitate. The organic layer was separated from the water layer by centrifugation (3500 rpm, 20 minutes). The plates containing the water and organic layer were incubated at -80 °C for 30 minutes to freeze the water layer and hence preventing accidental uptake of part of the water layer. 50 µl from the organic layer was transferred to a GC vial by a robotic pipetting station. 8 µl of the organic layer was injected on a gas chromatograph using an Astec CHIRALDEX G-TA column, isocratic 125 °C (Carrier gas He, 1,69 mL/min). Flame ionization detection: Retention time S-3a: 25.6 min, retention time R-3a 26.9 min. The assignment of the absolute configuration was based on earlier reported data^{15,22}.

### Expression and purification of enzymes

4-OT M45Y/F50A was purified according to a previously described purification procedure²⁰. Mutants I2M/L8Y/M45Y/F50A, I2W/L8Y/M45Y/F50A, L8F/M45Y/F50A and L8Y/M45Y/F50A were purified according to a modified protocol. The cell pellets were resuspended in 10 mM Tris pH 8 (instead of 10 mM sodium phosphate buffer). After loading on the DEAE-Sepharose column the column was washed 2x with 8 mL 10 mM Tris pH 8. 4-OT was eluted with 3x 8 mL of 10 mM sodium phosphate buffer pH 7.3. Typically the second and third fraction would contain pure 4-OT (>95% purity as determined by SDS PAGE). Fractions containing pure 4-OT were combined and concentrated using a vivaspin centrifugal concentrator (Sartorius, 5000 MWCO). After concentration to ∼3 mL, the sample was 2 times diafiltrated with 20mL 10 mM sodium phosphate buffer pH 7.3 to remove any traces of the Tris buffer. The concentration of purified 4-OT was determined by the Waddell method³⁵. The purified 4-OT was aliquoted, flash frozen in liquid nitrogen and stored at -80 °C until further use. All purified 4-OT mutants were analyzed by electron spray ionization mass spectrometry to confirm the correct mass of the protein.

The library I2X/L8X/M45Y/F50A was screened for the addition of **1** to **2a.** Activity and enantioselectivity of individual mutants were analyzed by gas chromatography. 960 clones were screened, and 5 clones were found to have an increased activity and strongly increased enantioselectivity, obtaining product *S***-3a** with an e.r. of at least 99:1. After sequencing of these clones, 2 different mutants were identified: I2W/L8Y/M45Y/F50A and I2M/L8Y/M45Y/F50A. These mutants were purified and tested to confirm their enantioselectivity. Both mutants catalyzed the addition of **1** to **2a** with very high enantioselectivity and product *S***-3a** was obtained with an e.r. of 99:1.

Since both positive hits from the screening of the I2X/L8X/M45Y/F50A library contain the L8Y mutation, mutant L8Y/M45Y/F50A was constructed to test the influence of mutations at position 2. Also, mutant L8F/M45Y/F50A was constructed to test the effect of a chemically highly similar residue at position 8 on catalytic properties. Both mutant L8Y/M45Y/F50A and mutant L8F/M45Y/F50A exhibited an excellent enantioselectivity obtaining **3a** with an e.r. of 99:1 (Table 1).

**Table 1: Enantioselectivity of 4-OT mutants for the addition of 1 to 2a^{a}.**

| | | | | |
|---|---|---|---|---|
| 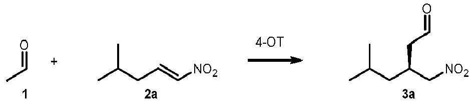 | | | | |

| **Entry** | **4-OT mutant** | **Co-solvent** | **e.r.^{b}** | **Abs. Conf.^{c}** |
|---|---|---|---|---|
| 1 | M45Y/F50A | 5 % DMSO | 90:10 | *S* |
| 2 | I2W/L8Y/M45Y/F50A | 5 % DMSO | 99:1 | *S* |
| 3 | I2M/L8Y/M45Y/F50A | 5 % DMSO | 99:1 | *S* |
| 4 | L8F/M45Y/F50A | 5 % DMSO | 99:1 | *S* |
| 5 | L8Y/M45Y/F50A | 5 % DMSO | 99:1 | *S* |
| 6 | L8Y/M45Y/F50A | 25 % EtOH | >99:1 | *S* |
| ^{a} Assay conditions: The reaction mixture consisted of 150 mM 1, 5 mM 2a, 100 µM of catalyst in 20 mM sodium phosphate buffer pH 6.5, 300 µl reaction volume. | | | | |
| ^{b} Determined by GC with a chiral stationary phase. | | | | |
| ^{c} Determined by literature comparison. | | | | |

Surprisingly, also the catalytic activity of mutant L8Y/M45Y/F50A for the addition of **1** to **2a** was considerably improved compared to our starting mutant M45Y/F50A (Fig. 2A). For the progress curve of the addition of 1 to 2a, the reaction mixtures consisted of the following: 5 mM 2a, DMSO (5% v/v), 100 µM 4-OT (2 mol% compared to 2a) in 20 mM sodium phosphate buffer pH 6.5, 500 µl final volume. The reaction was initiated by the addition of 1 to a final concentration of 150 mM, from a 1.5 M stock solution in 20 mM sodium phosphate buffer pH 6.5. Every 8 minutes a sample was withdrawn from the reaction mixture and a full spectrum was measured (200 nm to 500 nm). After the measurement, the sample was returned to the reaction mixture. The progress curve was constructed based on the absorbance at 249 nm, which corresponds to the λmax of 2a¹⁵. After 88 minutes, the reaction mixtures were extracted with 500µl toluene. The organic layer was separated from the inorganic layer by centrifugation. A sample from the organic layer was transferred to a GC vial and analyzed by GC with a chiral stationary phase (see library screening). No product could be identified for the reaction without enzyme.

Mutants I2M/L8Y/M45Y/F50A and I2W/L8Y/M45Y/F50A, although faster than M45Y/F50A, exhibited a lower catalytic rate than mutant L8Y/M45Y/F50A. Also mutant L8F/M45Y/F50A showed a lower catalytic rate compared to L8Y/M45Y/F50A. We tested the catalytic activity of mutant L8Y/M45Y/F50A under reported conditions for the addition of 1 to the aromatic substrate 2c to form 3c, the precursor for baclofen²².

For the progress curve of the addition of **1** to **2c,** the reaction mixtures consisted of the following: 1.3 mM **2c,** DMSO (45% v/v), 18µM 4-OT (1.4 mol% compared to **2c**) in 20 mM sodium phosphate buffer pH 6.5, 500µl final volume. The reaction was initiated by the addition of **1** to a final concentration of 65 mM from a 650 mM stock solution in sodium phosphate buffer pH 6.5. Every 8 minutes a sample was withdrawn from the reaction mixture and a full spectrum was measured (200 nm to 500 nm). After the measurement, the sample was returned to the reaction mixture. The progress curve was constructed based on the absorbance at 320 nm, which corresponds to the λmax of **2c**¹⁵.

To our delight, the catalytic rate for this reaction was also considerably improved compared to mutant M45Y/F50A (Fig. 2B). Coincidentally, mutant L8Y/M45Y/F50A proved to be much more stable in the presence of ethanol, tolerating ethanol concentrations of up to 30% v/v. The enantioselectivity of the L8Y/M45Y/F50A catalyzed addition of **1** to **2a** in the presence of 25% v/v ethanol was excellent, obtaining product *S***-3a** with an e.r. of >99:1 (see Table 1).

### Example 2: Enantioselective artificial Michaelase reaction at semi-preparative scale.

The synthetic usefulness of mutant L8Y/M45Y/F50A was further investigated in semi-preparative scale experiments with several nitroalkene acceptors (**2a-d**) (Table 2).

All semi preparative scale experiments were based on previously reported reaction conditions^{15,22}. Instead of DMSO, ethanol was used as a co-solvent, since 3a was obtained at the highest enantiopurity using 4-OT L8Y/M45Y/F50A in combination with ethanol as co-solvent.

For the synthesis of **3a** the reaction mixture consisted of the following: 5.0 mg **2a** (3 mM), 6.5 mg 4-OT L8Y/M45Y/F50A (75 µM, 2.5 mol% compared to **2a**), 2.56 mL ethanol (20% v/v) in 20 mM sodium phosphate buffer pH 6.5 to a final volume of 12.8 mL. The reaction was performed in a 25 mL Erlenmeyer flask sealed with a rubber stopper. The reaction was initiated by the addition of 108 µl of 1 (150 mM) and incubated at room temperature. At timely intervals a sample was withdrawn from the reaction mixture and the reaction progress was monitored by following the depletion of absorbance at 249 nm corresponding to the concentration **2a.** After the measurement, the sample was combined with the reaction mixture. After 50 minutes the reaction was finished and the reaction mixture was extracted 3x with 10 mL toluene. The organic layers were combined, washed with brine and dried over anhydrous Na₂SO₄. The dried organic layer was concentrated in vacuo, without any further purification to obtain **3a** (4.2 mg, 63% yield). The enantiopurity was determined by GC with a chiral stationary phase (see library screening).

For the synthesis of **3b-d** the reaction mixture consisted of the following: nitro alkene (17.9 mg **2b,** 2 mM; 14.3 mg **2c,** 1.3 mM; or 20.1 mg **2d,** 2 mM), 11.5 mg 4-OT L8Y/M45Y/F50A (28 µM, 1.4 mol% compared to **2b** and **2d,** 2.15 mol% compared to **2c**), ethanol (12 mL, 20% v/v for **2b** and **2d** and 18 mL, 30% v/v for **2c**) in 20 mM sodium phosphate buffer pH 6.5 to a final volume of 60 mL. The reaction was performed in a 100 mL Erlenmeyer flask sealed with a rubber stopper. The reaction was initiated by the addition of 169 µl 1 (50 mM) and incubated at room temperature. At timely intervals a sample was withdrawn from the reaction mixture and the reaction progress was monitored by following the depletion of absorbance (λₘₐₓ of **2b** and **2c** at 320 nm, λₘₐₓ of **2d** at 322 nm). After the measurement, the sample was combined with the reaction mixture. When the reaction was finished, the reaction mixture was extracted with 3x 40 mL ethyl acetate. The organic layers were combined, washed with brine and dried over anhydrous Na₂SO₄. The dried organic layer was concentrated in vacuo, without any further purification to obtain **3b-d** (**3b** 20.1 mg, 87% yield, **3c** 17.2 mg, 97% yield and **3d** 24.0 mg, 94% yield). The aldehyde functionality of **3b** and **3c** was derivatized to a cyclic acetal. The enantiopurity of **3b-d** was determined by reverse phase HPLC using chiralpak AD-RH column (150 mm x 4.6 mm, Daicel) (MeCN/water 70:30 for **3b,** MeCN/water 62:38 for **3c** and MeCN/water 30:70 for **3d,** 25 °C, 0.5 mL/min flow rate). Detection at 220 nm, retention time *R*-**3b**: 8.3 min, *S***-3b** 10.8 min, *R***-3c:** 29 min, *S***-3c** 37 min, *R*-**3d**: 39 min and *S***-3d** 41 min. The absolute configuration was determined by literature comparison¹⁵.

As is shown in Table 2, products **3a-d** could be synthesized with good yield (up to 97 %) and very high enantioselectivity (e.r up to >99:1). For results, see Table 2. In all cases, the pharmaceutically relevant enantiomer of the γ-nitroaldehyde was synthesized (*S***-3a** and *R*-**3b-d**). These data demonstrate the provision of a highly enantioselective artificial Michaelase with enhanced activity and improved solvent stability. Using this catalyst, precursors of 3 GABA analogs, pregabalin, baclofen and phenibut, can be synthesized with high yield and high enantiopurity.

**Table 2: Semi-preparative scale reactions of 4-OT L8Y/M45Y/F50A catalyzed Michael-type additions of 1 to 2a-d^{a}.**

| | | | | | | |
|---|---|---|---|---|---|---|
| 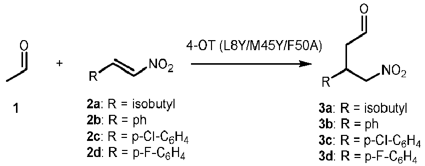 | | | | | | |

| **Entry** | **Product** | **4-OT (mol%)^{b}** | **Reaction time (min)** | **Co- solvent** | **Yield (%)** | **e.r.^{c}** |
|---|---|---|---|---|---|---|
| **1** | 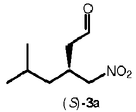 | 2.5 | 50 | 20% EtOH | 63 | >99:1 |
| **2** | 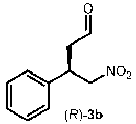 | 1.4 | 80 | 20% EtOH | 87 | 98:2 |
| **3** | 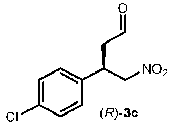 | 2.15 | 55 | 30% EtOH | 97 | >99:1 |
| **4** | 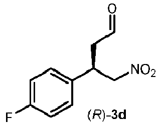 | 1.4 | 140 | 20% EtOH | 94 | >99:1 |
| ^{a} Assay conditions: Reaction mixtures consisted of 150 mM **1** (synthesis of **3a**), or 50 mM **1** (synthesis of **3b-d**), 3 mM **2a,** 1.3 mM **2c** or 2 mM **2b** and **2d** in 20 mM sodium phosphate buffer pH 6.5. The reaction volume was 12.8 mL (synthesis of **3a**) or 60 mL (synthesis of **3b-d**). | | | | | | |
| ^{b} Compared to the concentration nitroalkene. | | | | | | |
| ^{c} For **3a,** the e. r. was determined by GC with a chiral stationary phase. For **3b-d,** the product was converted into the corresponding ethylene glycol acetal and the e. r. was determind by HPLC with a chiral stationary phase. The absolute configuration was determined by literature comparison. | | | | | | |

### Example 3: Enzymatic two-step one-pot synthesis of γ-nitrocarboxylic acids.

Having constructed a mutant 4-OT enzyme that is highly enantioselective for *S***-3a** and *R*-**3b-d**, precursors for the pharmaceutically relevant enantiomer of several GABA analogs, we firstly focused our attention on the enzymatic oxidation of the aldehyde functionality to a carboxylate group. Not only would the resulting γ-nitrocarboxylic acids be one chemical step closer to the final GABA analogue, also, γ-nitrocarboxylic acids are more practical precursors, since carboxylate groups are less reactive than aldehydes.

To this end, an aldehyde dehydrogenase, PRO-ALD(003), was used. Initial experiments showed that PRO-ALD(003) was dependent on either NAD⁺ or NADP⁺ and could accept both *R***-3a** and *S***-3a.** For co-factor regeneration, we selected an NADH oxidase, PRO-NOX(009), which was shown to be strictly NADH dependent. Using our newly engineered artificial Michaelase, the mutant enzyme 4-OT L8Y/M45Y/F50A, in combination with PRO-ALD(003) and PRO-NOX(009) a biocatalytic two-step one-pot cascade was developed to synthesize *S***-4a** (Scheme 2). 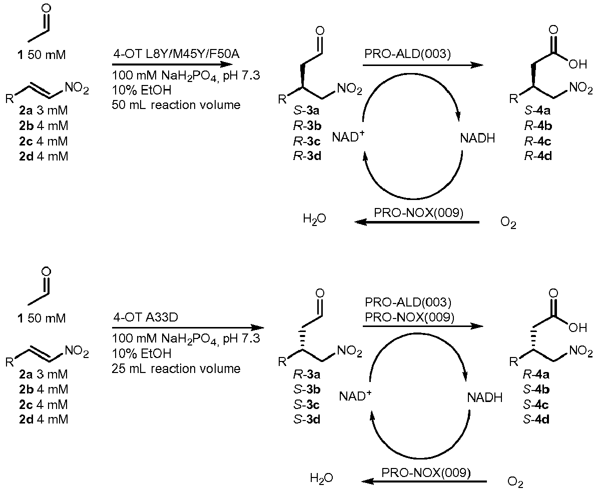 Scheme 2: Cascade synthesis γ-nitrocarboxylic acids. Michael-type addition of **1** to **2a-d** catalyzed by 4-OT L8Y/M45Y/F50A or 4-OT A33D to form either *S***-3a** and *R-***3b-d** or *R***-3a** and *S***-3b-d** respectively, followed by aldehyde oxidation catalyzed by PRO-ALD(003), using PRO-NOX(009) for cofactor recycling, to form either *S***-4a** and *R***-4b-d** or *R***-4a** and *S***-4b-d.**

The reaction mixture contained **2a** (22 mg, 3 mM), 4-OT L8Y/M45Y/F50A (5 mol% compared to **2a**), 5mL ethanol (10% v/v) in 0.1 M sodium phosphate buffer (pH 7.3) to a final volume of 50 mL. The reaction was initiated by the addition of **1** (2.5 mL from 1M stock, 50 mM) and incubated at room temperature. At timely intervals a sample was withdrawn from the reaction mixture and the reaction progress was monitored by following the depletion of absorbance at 249 nm corresponding to the concentration **2a.** After the measurement the sample was combined with the reaction mixture. After 20 minutes the reaction was finished and then to this reaction mixture PRO-ALD(003) (0.5 mg/mL), PRO-NOX(009) (1mg/mL) and NAD⁺ (8 mM) were added. After the reaction was completed, the reaction was stopped by acidifying the reaction to pH 5 and the reaction mixture was extracted with 3x 50 mL ethyl acetate. The organic layers were combined, washed with brine and dried over anhydrous Na₂SO₄. The dried organic layer was concentrated in vacuo and the crude mixture was purified by silica gel column chromatography (hexane/ethylacetate 4:1) to obtained **4a** (18mg, 64% yield). The acid functionality of **4a** was derivatized to the corresponding methyl ester. The same reaction procedures were followed for the synthesis of **4b-4d.** The enantiopurity of derivatized **4a** was determined by chiral-GC using a Hewlett Packard (HP) chiral 20% permethylated b-cyclodextrin column, 100 °C to 140 °C at a rate of 4 °C/min, then 140 °C to 160 °C at a rate of 2 °C/min and finally held at 160 °C. Flame ionization detection: retention time *R***-4a:** 16 min, *S***-4a** 17 min. The enantiopurity of derivatized **4b-d** were analyzed by reverse phase HPLC using chiral column (Chiralpak-ID, 150 mm x 4.6 mm, Daicel®) (MeCN/water 30:70, 25 °C, 1 mL/min flow rate). Detection at 210 nm, retention time *R*-**4b**: 22.6 min, *S***-4b:** 24.5 min, *R-***4c:** 19.4 min, *S***-4c:** 24.4 min, *R*-**4d**: 23.5 min and *S***-4d:** 26.6 min.

As is shown in Table 3, *S***-3a** was fully converted, and *S***-4a** was obtained with 64% isolated yield and with an excellent e.r. of 99:1. Using these conditions, also *R*-**4b-d** could be synthesized with good isolated yields (up to 71%) and excellent e.r.'s of 98:2 or higher. To demonstrate the modularity of the developed two-step cascade, instead of 4-OT L8Y/M45Y/F50A, we used the formerly reported 4-OT A33D as catalyst, which is enantioselective towards the opposite enantiomer²². Using 4-OT A33D as catalyst, also *R***-4a** and *S***-4b-d** could be obtained with good yields and excellent enantioselectivity (Table 3).

**Table 3: Two-step one-pot biocatalytic synthesis of γ-nitrocarboxylic acids 4a-d^{a}.**

| **Entry** | **Product** | **4-OT catalyst** | **Reaction time** | | **Isolate d yield (%)** | **e.r.^{e}** |
|---|---|---|---|---|---|---|
| | | | **Step I (min)^{c}** | **Step II (min)^{d}** | | |
| 1 | 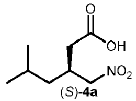 | L8Y/M45Y/F50A | **30** | **10** | **64** | **99:1** |
| 2 | 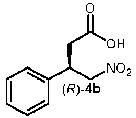 | L8Y/M45Y/F50A | **20** | **5** | **68** | **98:2** |
| 3 | 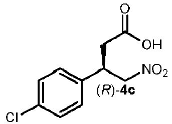 | L8Y/M45Y/F50A | **30** | **5** | **70** | **99:1** |
| 4 | 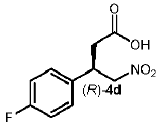 | L8Y/M45Y/F50A | **25** | **5** | **67** | **99:1** |
| 5 | 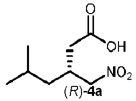 | A33D | **70** | **15** | **71** | **99:1** |
| 6 | 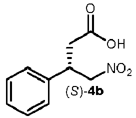 | A33D | **40** | **5** | **67** | **99:1** |
| 7 | 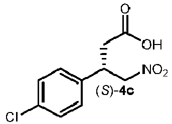 | A33D | **70** | **5** | **62** | **99:1** |
| 8 | 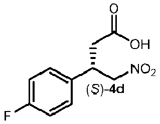 | A33D | **30** | **5** | **74** | **99:1** |
| ^{a} The reactions mixtures consisted of 50 mM **1**, 3 mM **2a** or 4 mM **2b-d** in 100 mM sodium phosphate buffer pH 7.3 and 10% v/v ethanol. 5 mol% of 4-OT (compared to concentration nitroalkene) was used, except for entry 6 (1.5 mol%) and entry 7 (3 mol%). PRO-ALD(003) was added to a final concentration of 0.5 mg/mL, PRO-NOX(009) was added to a final concentration of 1 mg/mL, the concentration NAD⁺ was 8 mM (**4a**) or 10 mM (**4b-d**). | | | | | | |
| ^{b} Compared to the concentration of nitroalkene. | | | | | | |
| ^{c} Monitored by UV spectroscopy. | | | | | | |
| ^{d} Monitored by HPLC. | | | | | | |
| ^{e} Products **4a-d** were esterificated and the e.r. was determined by GC or HPLC with a chiral stationary phase. | | | | | | |

### Example 4: Chemo-enzymatic three-step one-pot synthesis of GABA analogs

Finally, we focused on converting **4a-d** into **5a-d,** forming the final valuable GABA-analogs. To the best of our knowledge, no enzymes have been reported that can catalyze the full reduction to amine of nitro groups that are not directly attached to an aromatic group. Although many chemical nitro reduction procedures exist, most of these procedures use organic solvents and are not suitable to be performed in aqueous solutions. To prevent the necessity of intermediate work-up of **4a-d,** we selected nickel boride as catalyst (NiCl₂.6H₂O in combination with NaBH₄), since this has been reported to catalyze nitro reduction in aqueous solutions^{26,27}.

The reaction mixture contained nitroalkene (3mM of **2a** and 4mM of **2b-d**), 4-OT L8Y/M45Y/F50A (28-32 mg; 5 mol% compared to **2a-2d**), ethanol (10 % v/v) in 0.1 M NaH₂PO₄ buffer (pH 7.3) to a final volume of 25 mL. The reaction was initiated by the addition of **1** (1.25 mL from 1M stock in 0.1 M NaH₂PO₄ buffer pH 7.3, 50 mM) and incubated at room temperature. At timely intervals, a sample was withdrawn from the reaction mixture and the reaction progress was monitored by following the depletion in absorbance corresponding to the concentration nitroalkene. After the measurement the sample was combined with the reaction mixture. After the completion of first step reaction, to this the reaction mixture the aldehyde dehydrogenase PRO-ALD(003) (0.5 mg/mL), the NADH oxidase PRO-NOX009 (1mg/mL) and NAD⁺ (0.5 mM) were added. PRO-ALD(003) and PRO-NOX(009) were provided as crude cell-free extracts by Prozomix Ltd, and used without any further purification.

After 30 min, the reaction was quenched by acidifying the reaction mixture using 5 M HCl until the pH dropped to 3. To this, NiCl₂.6H₂O (40 mM) and NaBH₄ (40 mM) were added at 0 °C and stirring continued for 24h at room temperature. The reaction mixture was filtered through celite pad and filtrate was concentrated *in vacuo* and the resulting concentrated mixture was acidified to pH 3-4. The acidified reaction mixture was loaded on column packed with cation exchange resin (5g of Dowex® 50WX8 hydrogen form). After washing with deionized water (4 C.V.), the product was eluted out with 0.5 M (4 C.V.) - 1 M ammonia solution (4 C.V.).

The ninhydrin-positive fractions were collected and lyophilized to yield the products (**5a-5d**). **5a-d** were derivatized to diastereomers using sodium 2,4-dinitro-5-fluorophenyl-L-valine amide and the enantiopurity was determined by reverse phase HPLC using C18 column (Kinetex 5u EVO C18 100A, 150 mm x 4.6 mm, Phenomenex®) (25 °C, 1 mL/min flow rate). The mobile phase was 60:40 (v/v) mixture of aqueous buffer (0.2% triethylamine, pH adjusted to 3.5 with dilute orthophosphoric acid) and MeCN. Detection at 340 nm, retention time *S***-5a:** 16.1 min, *R*-**5a**: 21.4 min, *R*-**5b**: 10.9 min, *S***-5b:** 12.1 min, *R***-5c:** 17.2 min, *S***-5c:** 20.2 min, *R*-**5d**: 12.3 min and *S***-5d:** 13.6 min.

Indeed, nickel boride (NaBH₄/NiCl₂.6H₂O) was found to catalyze the reduction of **4a-d** to **5a-d.** Using this system, we set up a three-step one-pot cascade using 4-OT L8Y/M45Y/F50A to couple **1** to **2a** to form *S***-3a,** using PRO-ALD(003) in combination with PRO-NOX(009) to oxidize *S***-3a** to *S***-4a** and finally using NaBH₄/NiCl₂.6H₂O to reduce *S***-4a** to *S***-5a,** obtaining pregabalin (Scheme 3). 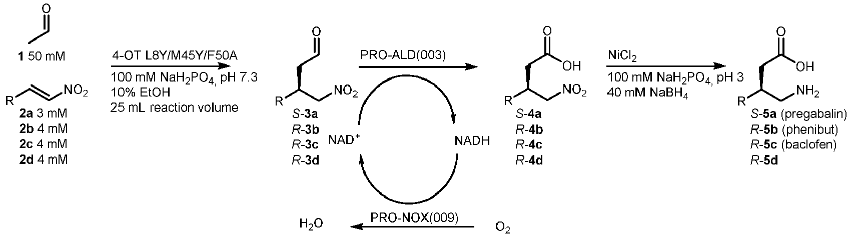 Scheme 3: Cascade synthesis of GABA analogs. Michael-type addition of **1** to **2a-d** catalyzed by 4-OT L8Y/M45Y/F50A to form *S***-3a** and *R*-**3b-d**, followed by aldehyde oxidation catalyzed by PRO-ALD(003), using PRO-NOX(009) for cofactor recycling, to form *S***-4a** and *R*-**4b-d**, followed by nitro reduction catalyzed by nickel boride to form *S***-5a** and *R*-**5b-d**.

This three-step one-pot cascade proved to be highly efficient obtaining **5a** with a final yield of 64% (86% yield per reaction step) and e.r. of 98:2. Similarly, this three-step one-pot cascade yielded various GABA analogs **5b-d,** in excellent yields of 70% up to 74% (89% up to 90% yield per reaction step) and with very high e.r.'s of 99:1 (Table 4).

**Table 4: Three-step one-pot chemoenzymatic synthesis of GABA analogue 5a-d^{a}.**

| **Entry** | **Product** | **Reaction time** | | | **Isolate d yield (%)^{e}** | **e.r.^{f}** |
|---|---|---|---|---|---|---|
| | | **step I (min)^{b}** | **step II (min)^{c}** | **step III (h)^{d}** | | |
| 1 | 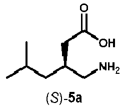 | **30** | **60** | **24** | **64** | **98:2** |
| 2 | 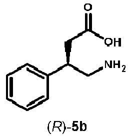 | **20** | **30** | **24** | **73** | **99:1** |
| 3 | 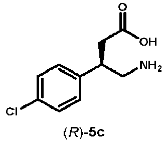 | **30** | **30** | **24** | **70** | **99:1** |
| 4 | 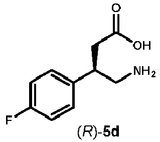 | **25** | **30** | **24** | **74** | **99:1** |
| ^{a} The reactions mixtures consisted of 50 mM **1**, 3 mM **2a** or 4 mM **2b-d** in 100 mM sodium phosphate buffer pH 7.3 and 10% v/v ethanol. 5 mol% of 4-OT (compared to concentration nitroalkene) was used; PRO-ALD(003) was added to a final concentration of 0.5 mg/mL; PRO-NOX(009) was added to a final concentration of 1 mg/mL; 0.5 mM of NAD⁺ was added. | | | | | | |
| ^{b} Monitored by UV spectroscopy. | | | | | | |
| ^{c} Monitored by HPLC. | | | | | | |
| ^{d} Monitored by TLC. | | | | | | |
| ^{e} Purified by cation exchange chromatography. | | | | | | |
| ^{f}Products **5a** - **5d** were derivatized using N_{α}-(2,4-Dinitro-5-fluorophenyl)-L-valinamide and the e.r. of the corresponding diastereomers was determined by HPLC with an achiral stationary phase; | | | | | | |

### REFERENCES

1. Winkler, C. K. et al. Chemoenzymatic asymmetric synthesis of pregabalin precursors via asymmetric bioreduction of β-cyanoacrylate esters using ene-reductases. J. Org. Chem. 78, 1525-1533 (2013).
2. Debarge, S. et al. Evaluation of several routes to advanced pregabalin intermediates: Synthesis and enantioselective enzymatic reduction using ene-reductases. Organic Process Research & Development 18, 109-121 (2014).
3. Hoekstra, M. S. et al. Chemical development of CI-1008, an enantiomerically pure anticonvulsant. Organic Process Research & Development 1, 26-38 (1997).
4. Gotoh, H., Ishikawa, H. & Hayashi, Y. Diphenylprolinol silyl ether as catalyst of an asymmetric, catalytic, and direct Michael reaction of nitroalkanes with α, 6-unsaturated aldehydes. Org. Lett. 9, 5307-5309 (2007).
5. Ordóñez, M., Cativiela, C. & Romero-Estudillo, I. An update on the stereoselective synthesis of γ-amino acids. Tetrahedron: Asymmetry 27, 999-1055 (2016).
6. Wiesner, M., Upert, G., Angelici, G. & Wennemers, H. Enamine catalysis with low catalyst loadings-high efficiency via kinetic studies. J. Am. Chem. Soc. 132, 6-7 (2009).
7. Wiesner, M., Revell, J. D., Tonazzi, S. & Wennemers, H. Peptide catalyzed asymmetric conjugate addition reactions of aldehydes to nitroethylene-a convenient entry into y2-amino acids. J. Am. Chem. Soc. 130, 5610-5611 (2008).
8. Wiesner, M., Revell, J. D. & Wennemers, H. Tripeptides as Efficient Asymmetric Catalysts for 1, 4-Addition Reactions of Aldehydes to Nitroolefins-A Rational Approach. Angewandte Chemie 120, 1897-1900 (2008).
9. Wiesner, M., Neuburger, M. & Wennemers, H. Tripeptides of the Type H-D-Pro-Pro-Xaa-NH2 as Catalysts for Asymmetric 1, 4-Addition Reactions: Structural Requirements for High Catalytic Efficiency. Chemistry-A European Journal 15, 10103-10109 (2009).
10. Zheng, Z., Perkins, B. L. & Ni, B. Diarylprolinol silyl ether salts as new, efficient, water-soluble, and recyclable organocatalysts for the asymmetric Michael addition on water. J. Am. Chem. Soc. 132, 50-51 (2009).
11. Qiao, Y., He, J., Ni, B. & Headley, A. D. Asymmetric Michael reaction of acetaldehyde with nitroolefins catalyzed by highly water-compatible organocatalysts in aqueous media. Advanced Synthesis & Catalysis 354, 2849-2853 (2012).
12. Geertsema, E. M. & Poelarends, G. J. in Science of Synthesis: Biocatalysis in Organic Synthesis 2 (eds Faber, K., Fessner, W. D. & Turner, N.) (Thieme Chemistry, 2014).
13. Garrabou, X., Verez, R. & Hilvert, D. Enantiocomplementary synthesis of γ-nitroketones using designed and evolved carboligases. J. Am. Chem. Soc. (2016).
14. Zandvoort, E., Geertsema, E. M., Baas, B., Quax, W. J. & Poelarends, G. J. Bridging between Organocatalysis and Biocatalysis: Asymmetric Addition of Acetaldehyde to β-Nitrostyrenes Catalyzed by a Promiscuous Proline-Based Tautomerase. Angewandte Chemie 124, 1266-1269 (2012).
15. Geertsema, E. M. et al. Biocatalytic Michael-Type Additions of Acetaldehyde to Nitroolefins with the Proline-Based Enzyme 4-Oxalocrotonate Tautomerase Yielding Enantioenriched γ-Nitroaldehydes. Chemistry-A European Journal 19, 14407-14410 (2013).
16. Miao, Y., Tepper, P. G., Geertsema, E. M. & Poelarends, G. J. Stereochemical Control of Enzymatic Carbon-Carbon Bond-Forming Michael-Type Additions by "Substrate Engineering". European journal of organic chemistry 2016, 5350-5354 (2016).
17. Whitman, C. P., Aird, B. A., Gillespie, W. R. & Stolowich, N. J. Chemical and enzymic ketonization of 2-hydroxymuconate, a conjugated enol. J. Am. Chem. Soc. 113, 3154-3162 (1991).
18. Stivers, J. T., Abeygunawardana, C., Mildvan, A. S., Hajipour, G. & Whitman, C. P. 4-Oxalocrotonate tautomerase: pH dependence of catalysis and p K a values of active site residues. Biochemistry (N. Y.) 35, 814-823 (1996).
19. Zandvoort, E., Geertsema, E. M., Quax, W. J. & Poelarends, G. J. Enhancement of the Promiscuous Aldolase and Dehydration Activities of 4-Oxalocrotonate Tautomerase by Protein Engineering. ChemBioChem 13, 1274-1277 (2012).
20. Zandvoort, E., Baas, B., Quax, W. J. & Poelarends, G. J. Systematic Screening for Catalytic Promiscuity in 4-Oxalocrotonate Tautomerase: Enamine Formation and Aldolase Activity. ChemBioChem 12, 602-609 (2011).
21. Poddar, H., Rahimi, M., Geertsema, E. M., Thunnissen, A. W. & Poelarends, G. J. Evidence for the formation of an enamine species during aldol and Michael-type addition reactions promiscuously catalyzed by 4-oxalocrotonate tautomerase. ChemBioChem 16, 738-741 (2015).
22. van der Meer, J. et al. Using mutability landscapes of a promiscuous tautomerase to guide the engineering of enantioselective Michaelases. Nature communications 7 (2016).
23. Carrea, G., Ottolina, G. & Riva, S. Role of solvents in the control of enzyme selectivity in organic media. Trends Biotechnol. 13, 63-70 (1995).
24. Klibanov, A. M. Enzymatic catalysis in anhydrous organic solvents. Trends Biochem. Sci. 14, 141-144 (1989).
25. Reetz, M. T., Bocola, M., Carballeira, J. D., Zha, D. & Vogel, A. Expanding the range of substrate acceptance of enzymes: combinatorial active-site saturation test. Angewandte Chemie International Edition 44, 4192-4196 (2005).
26. Khurana, J. M. & Gogia, A. Synthetically useful reactions with nickel boride. A review. Organic preparations and procedures international 29, 1-32 (1997).
27. Osby, J. O. & Ganem, B. Rapid and efficient reduction of aliphatic nitro compounds to amines. Tetrahedron Lett. 26, 6413-6416 (1985).
28. Muschiol, J. et al. Cascade catalysis-strategies and challenges en route to preparative synthetic biology. Chemical Communications 51, 5798-5811 (2015).
29. Hayashi, Y. Pot economy and one-pot synthesis. Chemical Science 7, 866-880 (2016).
30. Hayashi, Y., Sakamoto, D. & Okamura, D. One-Pot Synthesis of (S)-Baclofen via Aldol Condensation of Acetaldehyde with Diphenylprolinol Silyl Ether Mediated Asymmetric Michael Reaction as a Key Step. Org. Lett. 18, 4-7 (2015).
31. García-García, P., Ladepeche, A., Halder, R. & List, B. Catalytic asymmetric Michael reactions of acetaldehyde. Angewandte Chemie 120, 4797-4799 (2008).
32. Hayashi, Y., Itoh, T., Ohkubo, M. & Ishikawa, H. Asymmetric Michael reaction of acetaldehyde catalyzed by diphenylprolinol silyl ether. Angewandte Chemie International Edition 47, 4722-4724 (2008).
33. Jentzsch, K. I., Min, T., Etcheson, J. I., Fettinger, J. C. & Franz, A. K. Silyl fluoride electrophiles for the enantioselective synthesis of silylated pyrrolidine catalysts. J. Org. Chem. 76, 7065-7075 (2011).
34. Durchschein, K., Hall, M. & Faber, K. Unusual reactions mediated by FMN-dependent ene-and nitro-reductases. Green Chem. 15, 1764-1772 (2013).
35. Waddell, W. J. A simple ultraviolet spectrophotometric method for the determination of protein. J. Lab. Clin. Med. 48, 311-314 (1956).

## Claims

1. An isolated mutant 4-oxalocrototonate tautomerase (4-OT) enzyme comprising the following mutations
(i) leucine at position 8 substituted with a tyrosine (L8Y) or a phenylalanine (L8F);
(ii) methionine at position 45 substituted with a tyrosine (M45Y); and
(iii) phenylalanine at position 50 substituted with an alanine (F50A),
wherein the positions are numbered according to the amino acid sequence of 4-OT of *Pseudomonas putida.*

2. Mutant 4-OT according to claim 1, comprising the mutations L8Y, M45Y and F50A.

3. Mutant 4-OT according to claim 1 or 2, comprising an amino acid sequence selected from the group consisting of:
(a)PIAQIHI(**Y/F**)EGRSDEQKETLIREVSEAISRSLDAPLTSVRVIITE**Y**A KGH**A**GIGGELASKVRR
(b) an amino acid sequence which shows at least 95% sequence identity to (a), provided that it comprises the residues Y/F,Y and A indicated in bold at, respectively, positions 8, 45 and 50;
(c) an amino acid sequence which is shows at least 90% sequence identity to (a), provided that it comprises the residues Y/F,Y and A indicated in bold at, respectively, positions 8, 45 and 50; and
(d) PI(A/V)Q(I/L)(H/Y)(I/M)(Y/F)EGR(S/T/N)(D/N/G)(E/A)QKE(T/A/V/R) (L/F/M)IREVS (E/D/N)A(I/M)(S/V/A)R(S/A)L(D/G)AP(L/I/M)(T/A/P/E/D) (S/R/N)(V/I)RV(I/M)I(T/S/N)Y(A/P)(K/S)(G/A/S/N/V/T/A)HAGIGGE (L/P/S)A(S/K/R)(K/A/S)(V/I/L/T)(R/K/G/N/-)(R/G/P/-).

4. A nucleic acid molecule that encodes a mutant 4-OT enzyme according to any one of claims 1-3.

5. A recombinant vector that comprises the nucleic acid molecule of claim 4.

6. A recombinant host cell transformed with the vector of claim 5.

7. The recombinant host cell of claim 6 that is a bacterial, fungal or yeast host cell.

8. A method for the biocatalytic synthesis of a γ-nitroaldehyde via a Michael-type addition of an aldehyde to an α,β-unsaturated nitroalkene, comprising the use of a mutant 4-OT enzyme according to any one of claims 1-3, a nucleic acid according to claim 4, a vector according to claim 5 and/or a host cell according to claim 6 or 7.

9. A method for the synthesis of a precursor for the pharmaceutically relevant enantiomer of a GABA analog, comprising (i) providing a γ-nitroaldehyde according to a method of claim 8, followed by (ii) subjecting the thus obtained γ-nitroaldehyde to an enzymatic oxidation reaction catalyzed by an aldehyde dehydrogenase (EC 1.2.1.3), to obtain the corresponding γ-nitrocarboxylic acid.

10. A method for the enzymatic synthesis of a compound having a γ-nitrocarboxylic acid functionality, comprising subjecting a compound having a γ-nitroaldehyde functionality to an enzymatic oxidation reaction catalyzed by an aldehyde dehydrogenase (EC 1.2.1.3), to obtain the corresponding compound having a γ-nitrocarboxylic acid functionality.

11. Method according to claim 9 or 10, wherein said enzymatic oxidation reaction is performed in the presence of a co-factor regenerator, preferably wherein said co-factor regenerator is an NADH oxidase (EC 1.6.3.1).

12. Method according to any one of claims 9-11, wherein steps (i) and (ii) are performed as a two-step one-pot process.

13. Method according to any one of claims 9 and those depending thereon, further comprising step (iii) of chemically reducing the nitro-moiety of the γ-nitrocarboxylic acid to obtain a GABA analog.

14. Method according to claim 13, wherein said chemical reduction is performed in an aqueous solution, preferably using nickel boride (NaBH₄/NiCl₂.6H₂O) as catalyst.

15. Method according to claim 14, wherein steps (i), (ii) and (iii) are performed as a three-step one-pot process.

16. Method according to any one of claims 9-15, for the synthesis of a precursor of a GABA analog selected from the group consisting of pregabalin, baclofen, rolipram, phenibut and para-F-phenibut.
